# EUROPEAN PATENT SPECIFICATION

(11) **EP 1 496 742 B1**
(45) Date of publication and mention of the grant of the patent: **24.11.2010**
(21) Application number: 03745650.6
(22) Date of filing: 27.03.2003
(51) Int. Cl.: A61K 31/165, A61K 39/02, A61P 39/00, A61K 31/65, A61K 45/06

(54) **USE OF NON-ANTIBACTERIAL TETRACYCLINE ANALOGS AND FORMULATIONS THEREOF FOR THE TREATMENT OF BACTERIAL EXOTOXINS**
VERWENDUNG VON NICHTANTIBAKTERIELLEN TETRAZYKLIN-ANALOGA UND IHREN FORMULIERUNGEN FÜR DIE BEHANDLUNG VON BAKTERIELLEN EXOTOXINEN
UTILISATION D'ANALOGUES DE TETRACYCLINE NON-ANTIBACTERIENS ET LEURS FORMULATIONS DANS LE TRAITEMENT DES EXOTOXINES BACTERIENNES

(30) Priority: 29.03.2002 US 368478 P
(43) Date of publication of application: 19.01.2005
(60) Divisional application: 10174817.6
(73) Proprietor: THE RESEARCH FOUNDATION OF STATE UNIVERSITY OF NEW YORK, Albany, New York 12201-0009 (US)
(72) Inventor: GOLUB, Lorne, M., Smithtown, NY 11787 (US); WALKER, Stephen, G., East Setauket, NY 11733-2605 (US)
(74) Representative: Winckels, Johannes Hubertus F.
(86) International application number: PCT/US2003/009570
(87) International publication number: WO 2003/082011

(56) References cited:
- EP-A2- 0 256 713
- WO-A-00/72874
- DE-A1- 2 011 281
- GB-A- 995 032
- GB-A- 1 034 934
- US-A- 5 919 775
- US-A- 6 017 906
- US-A- 6 063 775
- US-B1- 6 245 735
- US-B1- 6 277 061
- US-B2- 6 384 081
- GREENWALD ET AL.: 'Biologic properties of non-antibiotic, chemically modified tetracyclines (CMTs): A structured, annotated bibliography' CURRENT & MEDICINAL CHEMISTRY vol. 8, 2001, pages 237 - 242, XP002970519

## Description

### BACKGROUND OF THE INVENTION

When bacteria attack a host, there is an incubation period during which there are mild or no symptoms. The incubation period varies among bacteria.

Once inside the host, some bacteria begin producing exotoxins. These exotoxins damage host tissue and organs, sometimes causing a sudden onset of hyperacute illness which progresses to shock, coma and death.

For example, an inhalation infection with *Bacillus anthracis* (anthrax) can have an incubation period of 3 to 60 days. Death from anthrax inhalation is considered inevitable if untreated, and probable in as many as 95% of treated cases if therapy is begun more than 48 hours after the onset of symptoms.

The lack of warning symptoms, sudden onset and almost absolute mortality, among other factors, have made anthrax a choice disease for use as a biological weapon of mass destruction. The threat of such a weapon has heightened research into modes of treatment and prevention of anthrax.

Of particular interest is a treatment for individuals at high risk of exposure, as well as for those who may have been exposed to anthrax, but are without symptoms. Currently, antibiotics such as ciprofloxacin are prescribed for such individuals. Due to the variably long incubation period with the inhaled form of anthrax, individuals potentially exposed are often put on an antibiotic therapy for sixty days.

Antibiotics target the bacteria itself. Often, in bacterial infections such as anthrax, the conventional therapy of antibiotics is administered too late. For example, ciprofloxacin has substantially no effect on the exotoxins released by the bacteria, which is the eventual cause of death. Therefore, once the infection has progressed to the point where sufficient exotoxin has been released, antibiotics alone have little or no effect. Even if the bacteria have been eliminated, remaining exotoxins may continue to cause tissue damage leading to death.

The problem with prescribing antibiotics as a treatment for individuals who may or may not be infected with bacteria such as anthrax is antibiotic resistance. The Center for Disease Control (CDC) has called antibiotic resistance one of the world's most pressing health problems. See, www.cdc.gov/antibioticresistance/. Thus, prescribing a sixty day course of antibiotics to potentially anthrax-exposed individuals increases the likelihood of antibiotic-resistant bacterial strains.

Hence, the prior art treatments for exotoxin-releasing bacterial infections, such as anthrax, are disadvantageous. Current treatments, such as administration of ciprofloxacin, do not target the deadly exotoxins or protect against tissue and organ damage. Moreover, a potentially unnecessary sixty day course of antibiotics may lead to antibiotic resistant bacterial strains.

An ideal treatment for exotoxin-releasing bacteria would be the targeting and neutralization, or disabling of the deadly exotoxins. Such treatment would provide protection against the exotoxins without using antibiotics until actual bacterial infection has been confirmed.

The compound, tetracycline is a member of a class of antibiotic compounds that is referred to as the tetracyclines, tetracycline compounds, tetracycline derivatives and the like. The compound tetracycline exhibits the following general structure:

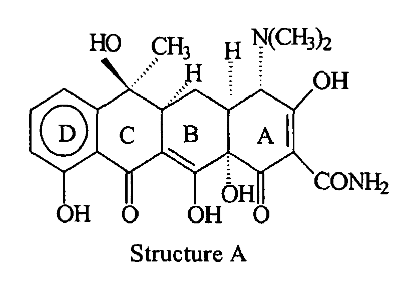

The numbering system of the tetracycline ring nucleus is as follows:

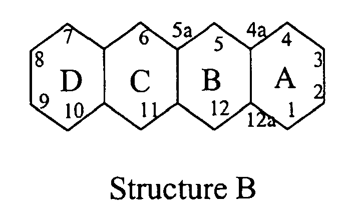

Tetracycline, as well as the terramycin and aureomycin derivatives, exist in nature, and are well known antibiotics. Natural tetracyclines may be modified without losing their antibiotic properties, although certain elements must be retained. The modifications that may and may not be made to the basic tetracycline structure have been reviewed by Mitscher in The Chemistry of Tetracyclines, Chapter 6, Marcel Dekker, Publishers, New York (1978). According to Mitscher, the substituents at positions 5-9 of the tetracycline ring system may be modified without the complete loss of antibiotic properties.

Changes to the basic ring system or replacement of the substituents at positions 4 and 10-12, however, generally lead to synthetic tetracyclines with substantially less or effectively no antimicrobial activity. Some examples of chemically modified non-antibacterial tetracyclines (hereinafter CMTs) are 4-dedimethylaminotetracyline, 4-dedimethylaminosancycline (6-demethyl-6-deoxy-4-dedimethylaminotetracycline), 4-dedimethylaminominocycline (7-dimethylamino-6-demethyl-6-deoxy-4-dedimethylaminotetracycline), and 4-dedimethylaminodoxycycline (5-hydroxy-6-deoxy-4-dedimethyaminotetracycline).

In addition to their antimicrobial properties, tetracyclines have been described as having a number of other uses. For example, tetracyclines are also known to inhibit the activity of collagen destructive enzymes produced by mammalian (including human) cells and tissues by non-antibiotic mechanisms. Such enzymes include the matrix metalloproteinases (MMPs), including collagenases (MMP-1, MMP-8 and MMP-13), gelatinases (MMP-2 and MMP-9), and others (e.g. MMP-12, MMP-14). See Golub et al., J. Periodont. Res. 20:12-23 (1985); Golub et al. Crit. Revs. Oral Biol. Med. 2:297-322 (1991); U.S. Patent Nos. 4,666,897; 4,704,383; 4,935,411; 4,9354,412. Also, tetracyclines have been known to inhibit wasting and protein degradation in mammalian skeletal muscle, U.S. Pat. No. 5,045,538, to inhibit inducible NO synthase, U.S. Patent Nos. 6,043,231 and 5,523,297, and phospholipase A₂, U.S. Patent Nos. 5,789,395 and 5,919,775, and to enhance IL-10 production in mammalian cells. These properties cause the tetracyclines to be useful in treating a number of diseases.

The object of this invention is to provide a method for protecting a mammal infected by, or at risk of exposure to, bacteria that produce exotoxins such as anthrax without the risk of antibiotic resistance.

### SUMMARY OF THE INVENTION

It has now been discovered that these and other objectives can be achieved by the following methods.

In a first embodiment of the invention, protection for a mammal at risk of acquiring a condition associated with bacteria that produce a calmodulin exotoxin, a metalloproteinase exotoxin, or both, is provided.

In a second embodiment, treatment for a mammal having a condition associated with bacteria that produce a calmodulin exotoxin, a metalloproteinase exotoxin, or both, is provided.

In a third embodiment, protection from acquiring a condition associated with B. anthracis for a mammal that has received or is scheduled to receive a vaccine against a bacteria that produces a calmodulin exotoxin, metalloproteinase exotoxin, or both, is provided.

In all embodiments, the bacteria is *Bacillus anthracis.*

In one embodiment the methods comprise administering to the mammal an effective amount of a non-antibacterial tetracycline, or a pharmaceutically acceptable salt thereof.

In another embodiment, the method further includes administration of an antibiotic, along with the non-antibacterial tetracycline.

In an additional embodiment the tetracycline is administered before a vaccine is administered, at the same time that a vaccine is administered, or after a vaccine is administered.

### DETAILED DESCRIPTION

The invention relates to treating conditions associated with a bacterial exotoxin with a tetracycline derivative, which is CMT-3 or CMT-308.

Examples of antibacterial tetracycline derivatives include tetracycline, as well as the 5-OH (oxytetracycline, e.g. terramycin^{™}) and 7-C1 (chlorotetracycline, e.g., aureomycin^{™}) derivatives, which exist in nature. Semisynthetic tetracyclines include, for example, doxycycline, minocycline and sancycline.

Some examples of antibacterial amounts of members of the tetracycline family include 100mg/day of doxycycline, 200mg/day of minocycline, 250mg of tetracycline four times a day, 1000mg/day of oxytetracycline, 600mg/day of demeclocycline and 600mg/day of lymecycline.

Examples of antibacterial tetracyclines administered in a non-antibacterial amount are depicted in Table 1, as follows:

**Table 1**

| Drug | Maximum Non-Antimicrobial Dose | Plasma Antimicrobial Threshold Level |
|---|---|---|
| Doxycycline | 20 mg b.i.d | 1.0 mcg/mL |
| Minocycline | 38 mg q.d. | 0.8 mcg/mL |
| Tetracycline | 60 mg q.d. | 0.5 mcg/mL |

Doxycycline administered at a 20 milligram dose twice daily is sold for the treatment of periodontal disease by CollaGenex Pharmaceuticals, Inc. of Newtown, Pennsylvania under the trademark Periostat ®.

In the invention, however, non-antibacterial tetracycline compounds selected from CMT-3 and CMT-308 are administered. For this embodiment, a class of compounds has been defined which are structurally related to the antibiotic tetracyclines, but which have had their antibiotic activity substantially or completely eliminated by chemical modification. Substantial elimination of antibiotic activity occurs when the antibiotic activity is ten times less than that of tetracycline, and preferably five times less than that of doxycycline.

One such group of chemically modified nonantibacterial tetracyclines (CMT's) includes any of the antibacterial 4-dedimethylaminotetracycline derivatives. Some examples of non-antibacterial tetracyclines include those compounds disclosed generically or specifically in co-pending U.S. patent application serial no. 09/573,654 filed on May 18, 2000.

Some examples of suitable 4-dedimethylaminotetracycline derivatives include the following general formulae (I) through (IV):

### General Formula (I)

### Structure A represents the 4-dedimethylaminosancycline (CMT-3) derivatives

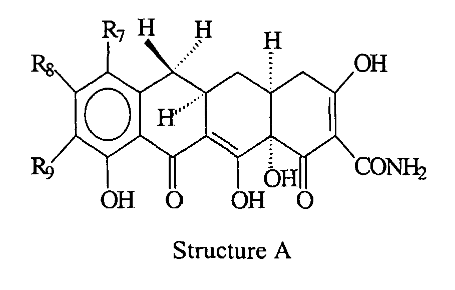

wherein R7, R8, and R9 taken together in each case, have the following meanings:

| | | | |
|---|---|---|---|
| | R7 | R8 | R9 |
| | azido | hydrogen | hydrogen |
| | dimethylamino | hydrogen | azido |
| | hydrogen | hydrogen | azido |
| | dimethylamino | hydrogen | amino |
| | acylamino | hydrogen | hydrogen |
| | amino | hydrogen | nitro |
| | hydrogen | hydrogen | (N,Ndimethyl)glycylamino |
| | amino | hydrogen | amino |
| | hydrogen | hydrogen | ethoxythiocarbonylthio |
| | dimethylamino | hydrogen | acylamino |
| | dimethylamino | hydrogen | diazonium |
| | dimethylamino | chloro | amino |
| | hydrogen | chloro | amino |
| | amino | chloro | amino |
| | acylamino | chloro | acylamino |
| | amino | chloro | hydrogen |
| | acylamino | chloro | hydrogen |
| | monoalkylamino | chloro | amino |
| | nitro | chloro | amino |
| | dimethylamino | chloro | acylamino |
| | dimethylamino | chloro | dimethylamino |
| | acylamino | hydrogen | hydrogen |
| | hydrogen | hydrogen | acylamino |
| (CMT-301) | bromo | hydrogen | hydrogen |
| (CMT-302) | nitro | hydrogen | hydrogen |
| (CMT-303) | hydrogen | hydrogen | nitro |
| (CMT-304) | acetamido | hydrogen | hydrogen |
| (CMT-305) | hydrogen | hydrogen | acetamido |
| (CMT-306) | hydrogen | hydrogen | dimethylamino |
| (CMT-307) | amino | hydrogen | hydrogen |
| (CMT-308) | hydrogen | hydrogen | amino |
| (CMT-309) | hydrogen | hydrogen | dimethylaminoacetamido |
| (CMT-310) | dimethylamino | hydrogen | hydrogen |
| (CMT-311) | hydrogen | hydrogen | palmitamide |

| | | | | |
|---|---|---|---|---|
| | R7 | R8 | R9 | R2 |
| (CMT-312) | hydrogen | hydrogen | hydrogen | CONHCH₂-pyrrolidin-1-yl |
| (CMT-313) | hydrogen | hydrogen | hydrogen | CONHCH₂-piperadin-1-yl |
| (CMT-314) | hydrogen | hydrogen | hydrogen | CONHCH₂-morpholin-1-yl |
| (CMT-315) | hydrogen | hydrogen | hydrogen | CONHCH₂-piperazin-1-yl |

### General Formula (II)

Structures B through E represent the 4-dedimethylaminodoxycycline (CMT-8) derivatives 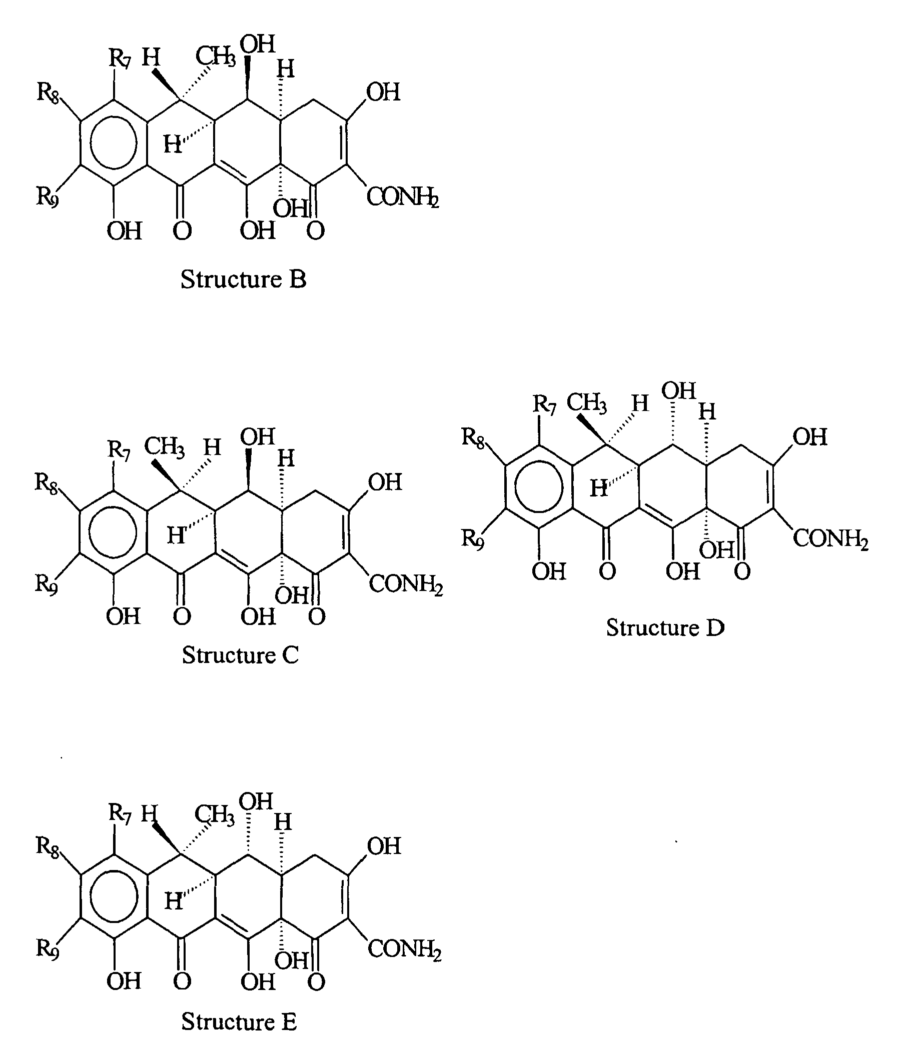 wherein R7, R8, and R9 taken together in each case, have the following meanings:

| | | | |
|---|---|---|---|
| | R7 | R8 | R9 |
| | azido | hydrogen | hydrogen |
| | dimethylamino | hydrogen | azido |
| | hydrogen | hydrogen | azido |
| | dimethylamino | hydrogen | amino |
| | acylamino | hydrogen | hydrogen |
| | hydrogen | hydrogen | acylamino |
| | amino | hydrogen | nitro |
| | hydrogen | hydrogen | (N,N-dimethyl)glycylamino |
| | amino | hydrogen | amino |
| | hydrogen | hydrogen | ethoxythiocarbonylthio |
| | dimethylamino | hydrogen | acylamino |
| | hydrogen | hydrogen | diazonium |
| | diazonium | hydrogen | hydrogen |
| | ethoxythiocarbonylthio | hydrogen | hydrogen |
| | dimethylamino | chloro | amino |
| | amino | chloro | amino |
| | acylamino | chloro | acylamino |
| | hydrogen | chloro | amino |
| | amino | chloro | hydrogen |
| | acylamino | chloro | hydrogen |
| | monoalkylamino | chloro | amino |
| | nitro | chloro | amino |
| (CMT-801) | hydrogen | hydrogen | acetamido |
| (CMT-802) | hydrogen | hydrogen | dimethylaminoacetamido |
| (CMT-803) | hydrogen | hydrogen | palmitamide |
| (CMT-804) | hydrogen | hydrogen | nitro |
| (CMT-805) | hydrogen | hydrogen | amino |
| (CMT-806) | hydrogen | hydrogen | dimethylamino |

R7 R8 R9 R2
(CMT-807) hydrogen hydrogen hydrogen CONHCH₂-pyrrolidin-1-yl (CMT-808) hydrogen hydrogen hydrogen CONHCH₂-piperadin-1-yl (CMT-809) hydrogen hydrogen hydrogen CONHCH₂-piperazine-1-yl

### General Formula (III)

Structure F represents the 4-dedimethylaminominocycline (CMT-10) derivatives 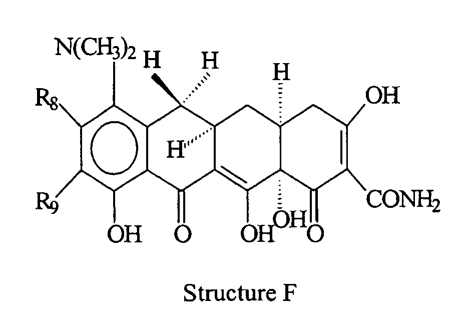 wherein R8 is hydrogen or halogen and R9 is selected from the group consisting of nitro (CMT-1002), (N,N-dimethyl)glycylamino, ethoxythiocarbonylthio. A compound related to structure F has a 7-trimethylammonium group instead of the 7-diemthylamino group, i.e. 7-trimethylammoniumsancycline (CMT-1001), and

### General Formula (IV)

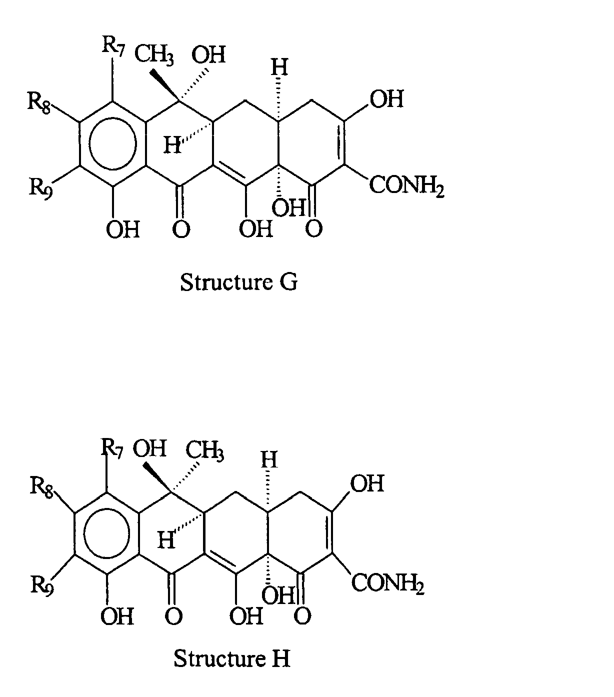

wherein R7, R8, and R9 taken together in each case, have the following meanings:

| R7 | R8 | R9 |
|---|---|---|
| amino | hydrogen | hydrogen |
| nitro | hydrogen | hydrogen |
| azido | hydrogen | hydrogen |
| dimethylamino | hydrogen | azido |
| hydrogen | hydrogen | amino |
| hydrogen | hydrogen | azido |
| hydrogen | hydrogen | nitro |
| bromo | hydrogen | hydrogen |
| dimethylamino | hydrogen | amino |
| acylamino | hydrogen | hydrogen |
| hydrogen | hydrogen | acylamino |
| amino | hydrogen | nitro |
| hydrogen | hydrogen | (N,N-dimethyl)glycylamino |
| amino | hydrogen | amino |
| diethylamino | hydrogen | hydrogen |
| hydrogen | hydrogen | ethoxythiocarbonylthio |
| dimethylamino | hydrogen | methylamino |
| dimethylamino | hydrogen | acylamino |
| dimethylamino | chloro | amino |
| amino | chloro | amino |
| acylamino | chloro | acylamino |
| hydrogen | chloro | amino |
| amino | chloro | hydrogen |
| acylamino | chloro | hydrogen |
| monoalkylamino | chloro | amino |
| nitro | chloro | amino |

Additional CMT's include, 4-dedimethylaminotetracycline (CMT-1), include tetracycline nitrile (CMT-2), 4-dedimethylaminochlorotetracycline (CMT-4), 4-dedimethylamino-4-hydroxytetracycline (CMT-6), 2a-dehydroxy-4-dedimethylaminotetracycline (CMT-7), and 1-deoxy-12a-dehydroxy-4-dedimethylaminotetracycline (CMT-9).

Non-antibacterial tetracycline compounds can be used in higher amounts than antibacterial tetracyclines, while avoiding the indiscriminate killing of bacteria, and the emergence of resistant bacteria. For example, 6-demethyl-6-deoxy-4-dedimethylaminotetracycline (CMT-3) may be administered in doses of about 10 to about 20mg/day which produces serum levels in humans of about 1µg/ml, or 40 to about 200mg/day, or in amounts that result in serum levels in humans of about 1.55µg/ml to about 10µg/ml.

The chemically modified tetracyclines can be made by methods known in the art. See, for example, Mitscher, L.A., The Chemistry of the Tetracycline Antibiotics, Marcel Dekker, New York (1978), Ch. 6, and U.S. Patents 4,704,383 and 5,532,227.

The invention also includes pharmaceutically acceptable salts of CMT-3 and CMT-308. The present invention embraces salts, including acid-addition and metal salts. Such salts are formed by well known procedures. By "pharmaceutically acceptable" is meant those salt-forming acids and metals which do not substantially contribute to the toxicity of the compound.

Some examples of suitable salts include salts of mineral acids such as hydrochloric, hydriodic, hydrobromic, phosphoric, metaphosphoric, nitric and sulfuric acids, as well as salts of organic acids such as tartaric, acetic, citric, malic, benzoic, glycollic, gluconic, gulonic, succinic, arylsulfonic, e.g. p-toluenesulfonic acids, and the like.

After preparation, the novel compounds of the present invention can be conveniently purified by standard methods known in the art. Some suitable examples include crystallization from a suitable solvent or partition-column chromatography.

The preferred pharmaceutical composition for use in the method of the invention includes a combination of the tetracycline compound in a suitable pharmaceutical carrier (vehicle) or excipient as understood by practitioners in the art. Examples of carriers and excipients include starch, milk, sugar, certain types of clay, gelatin, stearic acid or salts thereof, magnesium or calcium stearate, talc, vegetable fats or oils, gums and glycols.

The tetracycline compound may be administered to mammals by sustained release, as is known in the art. Sustained release administration is a method of drug delivery to achieve a certain level of the drug over a particular period of time. The level typically is measured by serum concentration.

The tetracycline compounds of the invention may be administered systemically. Systemic administration can be enteral or parenteral. Enteral administration is a preferred route of delivery of the tetracycline, and compositions including the tetracycline compound with appropriate diluents, carriers, and the like are readily formulated. Liquid or solid (e.g., tablets, gelatin capsules) formulations can be employed.

Administration can also be accomplished by a nebulizer or liquid mist. Nebulization is a preferred route of delivery of the tetracycline in situations where the respiratory system is particularly infected, for example, in the case of inhalation anthrax. By utilizing a nebulizer, the tetracycline is taken directly into the individuals respiratory system through inspiration.

Parenteral administration of the tetracycline compounds of the invention (e.g., intravenous, intramuscular, subcutaneous injection) is also contemplated. Formulations using conventional diluents, carriers, etc. such as are known in the art can be employed to deliver the compound.

Alternatively, delivery of the tetracycline compounds includes topical application. Topical administration is suitable in cutaneous infections such as, for example, cutaneous anthrax. Compositions deemed to be suited for such topical use include gels, salves, lotions, ointments and the like. Controlled release delivery of topical tetracyclines can be employed such as those currently used in dentistry such as ATRIDOX® (controlled release of topical doxycycline) and ARESTIN® (controlled release of topical minocycline).

The amount of tetracycline compound administered is any amount effective for reducing or inhibiting conditions associated with a bacterial exotoxin in the infected mammal. The actual preferred amounts of tetracycline compound in a specified case will vary according to the particular compositions formulated, the mode of application, and the particular subject being treated. The appropriate dose of the tetracycline compound can readily be determined by those skilled on the art.

The minimal amount of the tetracycline compound administered to a human is the lowest amount capable of providing effective treatment of the conditions. Effective treatment is a reduction or inhibition of the conditions, a reduction or inhibition of tissue destruction and/or prevention of death, of the mammal.

The maximal amount for a mammal is the highest amount that does not cause undesirable or intolerable side effects. Such doses can be readily determined by those skilled in the art. For example, CMTs can be systemically administered in a mammal in an amount of from about 0.05mg/kg/day to about 60mg/kg/day, and preferably from about 0.3mg/kg/day to about 18mg/kg/day. The practitioner is guided by skill and knowledge in the field, and the present invention includes, without limitation, dosages that are effective to achieve the desired antibacterial activity.

The appropriate dose of the tetracycline compound for topical administration can also be readily determined by those skilled in the art. For example, topical administration of CMTs in amounts of up to about 25% (w/w) in a vehicle can be administered without any toxicity in a human. Amounts from about 0.1% to about 10% are preferred.

The tetracyclines of the present invention protect mammals at risk of acquiring a condition associated with the bacterial exotoxins calmodulin and/or metalloproteinase. Calmodulin exotoxin, otherwise known as adenylate cyclase toxin, catalyzes the conversion of ATP to cAMP. In the claimed invention, calmodulin exotoxin is the edema factor (EF) of anthrax (caused by *Bacillus anthracis*).

Metalloproteinase exotoxin is a peptide hydrolase which uses a metal, such as zinc, in the catalytic mechanism. In the claimed invention, the lethal factor (LF) of anthrax is a zinc metalloproteinase exotoxin.

Many bacteria produce such exotoxins as part of their life cycle. For example, *Clostridium perfringens, Bordetella pertussis, Bacteriodes fragilis* and *Pseudomonas aeruginosa* are bacteria other than *Bacillus anthracis* that produce exotoxins.

A mammal which can benefit from the present invention could be any mammal. Categories of mammals include, for example, humans, farm animals, domestic animals, laboratory animals, etc. Some examples of farm animals include cows, pigs, horses, goats, etc. Some examples of domestic animals include dogs, cats, etc. Some examples of laboratory animals include rats, mice, rabbits, guinea pigs, etc.

A mammal at risk of acquiring a condition associated with a bacteria that produces a calmodulin exotoxin or a metalloproteinase exotoxin, or both, includes mammals that have been, are suspected of having been, or are expected to be exposed to, or infected with, a bacteria that produces such exotoxins. Mammals that may have been exposed to a bacteria that produces a calmodulin exotoxin or a metalloproteinase exotoxin, or both, include, for example, military personnel, individuals that handle animal skins, individuals that live in endemic areas, health care professionals who may treat or have treated infected individuals, and individuals that have been in contact with, or in the vicinity of, an area that has tested positive for the presence of such bacteria.

The invention can also include administration of an antibiotic, such as ciprofloxacin or doxycycline, to an individual having a condition associated with bacteria that produce a calmodulin exotoxin, a metalloproteinase exotoxin, or both, along with the tetracycline. The antibiotic can be administered before, concurrently, or after the tetracycline is administered.

In one embodiment, the mammal may have received, or may be scheduled to receive, a vaccine against a bacteria that produces a calmodulin or metalloproteinase exotoxin, or both. The tetracyclines of the present invention can be administered before, during or after a vaccine against a bacteria that produces a calmodulin or metalloproteinase exotoxin, or both is administered.

The tetracyclines of the present invention provide a host with protection against conditions associated with the metalloproteinase and/or calmodulin bacterial exotoxins. Conditions associated with these bacterial exotoxins include the reactions that occur once a bacterium enters the host.

Examples of such conditions include hemolysis, inhibition of protein synthesis, flaccid paralysis, spastic paralysis, emesis, inflammation, fever, shock, localized erythematous reactions, tissue destruction, diarrhea and other conditions as are known in the art, including death.

## Claims

1. CMT-3 or CMT-308, or a pharmaceutically acceptable salt thereof, for use in protecting a mammal at risk of acquiring a condition associated with *Bacillus anthracis* wherein an effective amount of said CMT-3 or CMT-308, or a pharmaceutically acceptable salt thereof is to be administered to the mammal.

2. CMT-3 or CMT-308, or a pharmaceutically acceptable salt thereof, for use in treating a mammal having a condition associated with *Bacillus anthracis,* wherein an effective amount of said CMT-3 or CMT-308, or a pharmaceutically acceptable salt thereof is to be administered to the mammal.

3. CMT-3 or CMT-308, or a pharmaceutically acceptable salt thereof for use according to claim 2, wherein further an antibiotic is to be administered.

4. CMT-3 or CMT-308, or a pharmaceutically acceptable salt thereof for use according to claim 3, wherein the antibiotic is ciprofloxacin.

5. CMT-3 or CMT-308, or a pharmaceutically acceptable salt thereof for use according to claim 3, wherein the antibiotic is doxycycline.

6. CMT-3 or CMT-308, or a pharmaceutically acceptable salt thereof, for use in protecting a mammal that has received or is scheduled to receive a vaccine against *Bacillus anthracis,* from acquiring a condition associated with Bacillus anthracis, wherein an effective amount of said CMT-3 or CMT-308, or a pharmaceutically acceptable salt thereof is to be administered to the mammal.

7. CMT-3 or CMT-308, or a pharmaceutically acceptable salt thereof, for use according to claim 6, wherein the CMT-3 or CMT-308, or the pharmaceutically acceptable salt thereof, is to be administered before the vaccine is administered.

8. CMT-3 or CMT-308, or a pharmaceutically acceptable salt thereof, for use according to claim 6 wherein the CMT-3 or CMT-308, or the pharmaceutically acceptable salt thereof, is to be administered at the same time that the vaccine is administered.

9. CMT-3 or CMT-308, or a pharmaceutically acceptable salt thereof, for use according to claim 6, wherein the CMT-3 or CMT-308, or the pharmaceutically acceptable salt thereof, is to be administered after the vaccine is administered.

10. Use of CMT-3 or CMT-308, or a pharmaceutically acceptable salt thereof, for the preparation of a medicament for protecting a mammal at risk of acquiring a condition associated with *Bacillus anthracis*, wherein an effective amount of said CMT-3 or CMT-308, or a pharmaceutically acceptable salt thereof is to be administered to the mammal.

11. Use of CMT-3 or CMT-308, or a pharmaceutically acceptable salt thereof, for the preparation of a medicament for treating a mammal having a condition associated with *Bacillus anthracis,* wherein an effective amount of said CMT-3 or CMT-308, or a pharmaceutically acceptable salt thereof is to be administered to the mammal.

12. Use according to claim 11, wherein further anantibiotic is to be administered.

13. Use according to claim 12, wherein the antibiotic is ciprofloxacin.

14. Use according to claim 12, wherein the antibiotic is doxycycline.

15. Use of CMT-3 or CMT-308, or a pharmaceutically acceptable salt thereof, for the preparation of a medicament for protecting a mammal that has received or is scheduled to receive a vaccine against *Bacillus anthracis,* from acquiring a condition associated with Bacillus anthracis, wherein an effective amount of said CMT-3 or CMT-308, or a pharmaceutically acceptable salt thereof is to be administered to the mammal.

16. Use according to claim 15, wherein the CMT-3 or CMT-308, or the pharmaceutically acceptable salt thereof, is to be administered before the vaccine is administered.

17. Use according to claim 15, wherein the CMT-3 or CMT-308, or the pharmaceutically acceptable salt thereof, is to be administered at the same time that the vaccine is administered.

18. Use according to claim 15, wherein the CMT-3 or CMT-308, or the pharmaceutically acceptable salt thereof, is to be administered after the vaccine is administered.

## Patentansprüche

1. CMT-3 oder CMT-308 oder ein pharmazeutisch annehmbares Salz davon zur Verwendung beim Schützen eines Säugetiers, welches gefährdet ist, einen Zustand, welcher mit *Bazillus anthracis* assoziiert ist, zu erlangen, wobei eine wirksame Menge des CMT-3 oder CMT-308 oder eines pharmazeutisch annehmbaren Salzes davon an das Säugetier zu verabreichen ist.

2. CMT-3 oder CMT-308 oder ein pharmazeutisch annehmbares Salz davon zur Verwendung beim Behandeln eines Säugetiers, welches an einem Zustand, welcher mit *Bazillus anthracis* assoziiert ist, leidet, wobei eine wirksame Menge des CMT-3 oder CMT-308 oder eines pharmazeutisch annehmbaren Salzes davon an das Säugetier zu verabreichen ist.

3. CMT-3 oder CMT-308 oder ein pharmazeutisch annehmbares Salz davon zur Verwendung gemäß Anspruch 2, wobei weiter ein Antibiotikum zu verabreichen ist.

4. CMT-3 oder CMT-308 oder ein pharmazeutisch annehmbares Salz davon zur Verwendung gemäß Anspruch 3, wobei das Antibiotikum Ciprofloxacin ist.

5. CMT-3 oder CMT-308 oder ein pharmazeutisch annehmbares Salz davon zur Verwendung gemäß Anspruch 3, wobei das Antibiotikum Doxycyclin ist.

6. CMT-3 oder CMT-308 oder ein pharmazeutisch annehmbares Salz davon zur Verwendung beim Schützen eines Säugetiers, welches ein Vakzin gegen *Bazillus anthracis* erhalten hat, oder dafür vorgesehen ist, vor dem Erlangen eines Zustands, welcher mit *Bazillus anthracis* assoziiert ist, wobei eine wirksame Menge des CMT-3 oder CMT-308 oder eines pharmazeutisch annehmbaren Salzes davon an das Säugetier zu verabreichen ist.

7. CMT-3 oder CMT-308 oder ein pharmazeutisch annehmbares Salz davon zur Verwendung gemäß Anspruch 6, wobei das CMT-3 oder CMT-308 oder das pharmazeutisch annehmbare Salz davon zu verabreichen ist, bevor das Vakzin verabreicht wird.

8. CMT-3 oder CMT-308 oder ein pharmazeutisch annehmbares Salz davon zur Verwendung gemäß Anspruch 6, wobei das CMT-3 oder CMT-308 oder das pharmazeutisch annehmbare Salz davon zur gleichen Zeit, zu der das Vakzin verabreicht wird, zu verabreichen ist.

9. CMT-3 oder CMT-308 oder ein pharmazeutisch annehmbares Salz davon zur Verwendung gemäß Anspruch 6, wobei das CMT-3 oder CMT-308 oder das pharmazeutisch annehmbare Salz davon zu verabreichen ist, nachdem das Vakzin verabreicht ist.

10. Verwendung von CMT-3 oder CMT-308 oder einem pharmazeutisch annehmbaren Salz davon zur Herstellung eines Medikaments zum Schützen eines Säugetiers, welches gefährdet ist, einen Zustand, welcher mit *Bazillus anthracis* assoziiert ist, zu erlangen, wobei eine wirksame Menge des CMT-3 oder CMT-308 oder eines pharmazeutisch annehmbaren Salzes davon an das Säugetier zu verabreichen ist.

11. Verwendung von CMT-3 oder CMT-308 oder einem pharmazeutisch annehmbaren Salz davon zur Herstellung eines Medikaments zum Behandeln eines Säugetiers, welches an einem Zustand, welcher mit *Bazillus anthracis* assoziiert ist, leidet, wobei eine wirksame Menge des CMT-3 oder CMT-308 oder eines pharmazeutisch annehmbaren Salzes davon an das Säugetier zu verabreichen ist.

12. Verwendung gemäß Anspruch 11, wobei weiter ein Antibiotikum zu verabreichen ist.

13. Verwendung gemäß Anspruch 12, wobei das Antibiotikum Ciprofloxacin ist.

14. Verwendung gemäß Anspruch 12, wobei das Antibiotikum Doxycyclin ist.

15. Verwendung von CMT-3 oder CMT-308 oder einem pharmazeutisch annehmbaren Salz davon zur Herstellung eines Medikaments zum Schützen eines Säugetiers, welches ein Vakzin gegen *Bazillus anthracis* erhalten hat oder dafür vorgesehen ist, vor dem Erlangen eines Zustands, welcher mit *Bazillus anthracis* assoziiert ist, wobei eine wirksame Menge des CMT-3 oder CMT-308 oder eines pharmazeutisch annehmbaren Salzes davon an das Säugetier zu verabreichen ist.

16. Verwendung gemäß Anspruch 15, wobei das CMT-3 oder CMT-308 oder das pharmazeutisch annehmbare Salz davon zu verabreichen ist, bevor das Vakzin verabreicht wird.

17. Verwendung gemäß Anspruch 15, wobei das CMT-3 oder CMT-308 oder das pharmazeutisch annehmbare Salz davon zur gleichen Zeit, zu der das Vakzin verabreicht wird, zu verabreichen ist.

18. Verwendung gemäß Anspruch 15, wobei das CMT-3 oder CMT-308 oder das pharmazeutisch annehmbare Salz davon zu verabreichen ist, nachdem das Vakzin verabreicht ist.

## Revendications

1. CMT-3 ou CMT-308 ou sel pharmaceutiquement acceptable de ces derniers, pour utilisation dans la protection d'un mammifère risquant de contracter une pathologie associée à *Bacillus anthracis* où une quantité efficace dudit CMT-3 ou CMT-308 ou d'un sel pharmaceutiquement acceptable de ces derniers doit être administrée au mammifère.

2. CMT-3 ou CMT-308 ou sel pharmaceutiquement acceptable de ces derniers pour utilisation dans le traitement d'un mammifère ayant une pathologie associée à *Bacillus anthracis* où une quantité efficace dudit CMT-3 ou CMT-308 ou d'un sel pharmaceutiquement acceptable de ces derniers doit être administrée au mammifère.

3. CMT-3 ou CMT-308 ou sel pharmaceutiquement acceptable de ces derniers pour utilisation selon la revendication 2, où un antibiotique doit être en outre administré.

4. CMT-3 ou CMT-308 ou sel pharmaceutiquement acceptable de ces derniers pour utilisation selon la revendication 3, où l'antibiotique est la ciprofloxacine.

5. CMT-3 ou CMT-308 ou sel pharmaceutiquement acceptable de ces derniers selon la revendication 3, où l'antibiotique est la doxycycline.

6. CMT-3 ou CMT-308 ou sel pharmaceutiquement acceptable de ces derniers pour utilisation dans la protection d'un mammifère ayant reçu ou devant recevoir un vaccin contre *Bacillus anthracis* contre la contraction d'une pathologie associée à *Bacillus anthracis* où une quantité efficace dudit CMT-3 ou CMT-308 ou d'un sel pharmaceutiquement acceptable de ces derniers doit être administrée au mammifère.

7. CMT-3 ou CMT-308 ou sel pharmaceutiquement acceptable de ces derniers pour utilisation selon la revendication 6, où CMT-3 ou CMT-308 ou le sel pharmaceutiquement acceptable de ces derniers doit être administré avant l'administration du vaccin.

8. CMT-3 ou CMT-308 ou sel pharmaceutiquement acceptable de ces derniers pour utilisation selon la revendication 6, où CMT-3 ou CMT-308 ou le sel pharmaceutiquement acceptable de ces derniers doit être administré au moment de l'administration du vaccin.

9. CMT-3 ou CMT-308 ou sel pharmaceutiquement acceptable de ces derniers pour utilisation selon la revendication 6, où CMT-3 ou CMT-308 ou le sel pharmaceutiquement acceptable de ces derniers doit être administré après l'administration du vaccin.

10. Utilisation de CMT-3 ou CMT-308 ou sel pharmaceutiquement acceptable de ces derniers pour la préparation d'un médicament destiné à protéger un mammifère risquant de contracter une pathologie associée à *Bacillus anthracis,* où une quantité efficace dudit CMT-3 ou CMT-308 ou d'un sel pharmaceutiquement acceptable de ces derniers doit être administrée au mammifère.

11. Utilisation de CMT-3 ou CMT-308 ou sel pharmaceutiquement acceptable de ces derniers pour la préparation d'un médicament destiné à traiter un mammifère ayant une pathologie associée à *Bacillus anthracis,* où une quantité efficace dudit CMT-3 ou CMT-308 ou d'un sel pharmaceutiquement acceptable de ces derniers doit être administrée au mammifère.

12. Utilisation selon la revendication 11, où un antibiotique doit être en outre administré.

13. Utilisation selon la revendication 12, où l'antibiotique est la ciprofloxacine.

14. Utilisation selon la revendication 12, où l'antibiotique est la doxycycline.

15. Utilisation de CMT-3 ou CMT-308 ou d'un sel pharmaceutiquement acceptable de ces derniers pour la préparation d'un médicament destiné à protéger un mammifère ayant reçu ou devant recevoir un vaccin contre *Bacillus anthracis* contre la contraction d'une pathologie associée à *Bacillus anthracis,* où une quantité efficace dudit CMT-3 ou CMT-308 ou d'un sel pharmaceutiquement acceptable de ces derniers doit être administrée au mammifère.

16. Utilisation selon la revendication 15, où CMT-3 ou CMT-308 ou le sel pharmaceutiquement acceptable de ces derniers doit être administré avant l'administration du vaccin.

17. Utilisation selon la revendication 15, où CTM-3 ou CMT-308 ou le sel pharmaceutiquement acceptable de ces derniers doit être administré au moment de l'administration du vaccin.

18. Utilisation selon la revendication 15, où CTM-3 ou CMT-308 ou le sel pharmaceutiquement acceptable de ces derniers doit être administré après l'administration du vaccin.
